# EUROPEAN PATENT APPLICATION

(11) **EP 4 789 656 A1**
(43) Date of publication of application: **12.08.2026**
(21) Application number: 25155911.8
(22) Date of filing: 05.02.2025
(51) Int. Cl.: A61K 8/365, A61K 8/42, A61K 8/60, A61Q 5/00, A61Q 5/06, A61Q 5/12

(54) **METHOD FOR INCREASING THE ELASTICITY OF KERATIN FIBERS**

(71) Applicant: Beautyge, S.L., 08940 Cornella de Llobregat (ES)
(72) Inventor: BORREDA MORAN, Andrea, 08940 CORNELLA DE LLOBREGAT (ES); COLOM VIVES, Olga, 08940 CORNELLA DE LLOBREGAT (ES); MURO URANGA, Ander, 08940 CORNELLA DE LLOBREGAT (ES); VALLECILLOS LOPEZ, Rocio, 08940 CORNELLA DE LLOBREGAT (ES)
(74) Representative: Gallardo, Antonio M.

(57) **Abstract**

A method for increasing the elasticity of keratin fibers is described. The method for increasing the elasticity of keratin fibers comprises the steps of:
(i) treating the keratin fibers by topical application of a cosmetic composition comprising, in a cosmetically acceptable medium:
(a) a mixture of Hydroxypropylgluconamide and Hydroxypropylammonium Gluconate; and
(b) lactic acid, or any cosmetically acceptable salt thereof;

(ii) allowing the composition to remain in contact with the keratin fibers for a period ranging from about 5 seconds to about 1 hour.
This method increases the elasticity of keratin fibers, in particular human hair, even in the case of damaged hair, reducing its trend to breakage under mechanical stress such as brushing or combing and hence, increasing its cosmetic appearance.

## Description

### TECHNICAL FIELD

A method for increasing the elasticity of keratin fibers is described. This method increases the elasticity of keratin fibers, in particular human hair, even in the case of damaged hair, reducing its trend to breakage under mechanical stress such as brushing or combing and hence, increasing its cosmetic appearance.

### BACKGROUND

The information provided below is not admitted to be prior art to the present disclosure, but is provided solely to assist the understanding of the reader.

Hair elasticity is an important characteristic of hair that determines its overall health and strength. It refers to the ability of hair strands to stretch and return to their original length without breaking or snapping. In simpler terms, it is the measure of how much hair can withstand tension before reaching its breaking point.

Hair elasticity is crucial as it allows consumers to style, brush, and manipulate their hair without causing damage or breakage.

Several factors can affect hair's elasticity, including genetics, age, and external factors. Some people are born with naturally high or low elasticity due to their genetics. As we age, our hair also tends to lose some of its elasticity due to a decrease in collagen production.

External factors such as heat styling, chemical treatments, and environmental stressors like pollution and UV rays can also impact hair elasticity. Overexposure to these elements can damage the protein structure of the hair and lead to low elasticity.

Therefore, there is a need for hair treatments that can provide an increase (improvement) in the elasticity of hair, in particular in damaged hair.

### SUMMARY

Described herein is a method for increasing the elasticity of keratin fibers comprising the steps of:
(i) treating the keratin fibers by topical application of a cosmetic composition comprising, in a cosmetically acceptable medium:
   (a) a mixture of Hydroxypropylgluconamide and Hydroxypropylammonium Gluconate; and
   (b) lactic acid, or any cosmetically acceptable salt thereof;
(ii) allowing the composition to remain in contact with the keratin fibers for a period ranging from about 5 seconds to about 1 hour.

This method increases the elasticity of keratin fibers, in particular human hair, even in the case of damaged hair, reducing its trend to breakage under mechanical stress such as brushing or combing and hence, increasing its cosmetic appearance.

In an embodiment, in the method described herein, in step (ii) the composition is allowed to remain in contact with the keratin fibers for a period ranging from about 10 seconds to about 45 minutes.

In an embodiment, in the cosmetic composition for use in step (i) of the method described herein, the molar ratio of Hydroxypropylgluconamide to Hydroxypropylammonium Gluconate in component (a) ranges from about 20:80 to about 80:20.

In an embodiment, in the cosmetic composition for use in step (i) of the method described herein, the component (b) comprises L-lactic acid, or any cosmetically acceptable salt thereof.

In an embodiment, in the cosmetic composition for use in step (i) of the method described herein, the total amount of the component (a) is in the range of about 0.05 to about 10 wt.%, based on the total weight of the composition.

In an embodiment, in the cosmetic composition for use in step (i) of the method described herein, the total amount of the component (b) is in the range of about 0.2 to about 10 wt.%, based on the total weight of the composition.

In an embodiment, the cosmetic composition for use in step (i) of the method described herein comprises, in a cosmetically acceptable medium, based on the total weight of the composition:
(a) about 0.05-10 wt.% of a mixture of Hydroxypropylgluconamide and Hydroxypropylammonium Gluconate; and
(b) about 0.2-10 wt.% of lactic acid, or any cosmetically acceptable salt thereof.

In an embodiment, the cosmetic composition for use in step (i) of the method described herein comprises, in a cosmetically acceptable medium, based on the total weight of the composition:
(a) about 0.1-7.5 wt.% of a mixture of Hydroxypropylgluconamide and Hydroxypropylammonium Gluconate; and
(b) about 0.3-7.5 wt.% of lactic acid, or any cosmetically acceptable salt thereof.

In an embodiment, the cosmetic composition for use in step (i) of the method described herein comprises, in a cosmetically acceptable medium, based on the total weight of the composition:
(a) about 0.2-7.5 wt.% of a mixture of Hydroxypropylgluconamide and Hydroxypropylammonium Gluconate, wherein the molar ratio of Hydroxypropylgluconamide to Hydroxypropylammonium Gluconate in component (a) ranges from about 20:80 to about 80:20; and
(b) about 0.5-7.5 wt.% of L-lactic acid, or any cosmetically acceptable salt thereof.

In an embodiment, in the method described herein, the keratin fibers are human hair.

In an embodiment, in the method described herein, human hair has been previously damaged as a result of at least one treatment selected from hair bleaching, hair dyeing, hair straightening, hair permanent wave (hair perm), blow-drying, or combinations thereof.

In an embodiment, the cosmetic composition for use in step (i) of the method described herein has a pH in the range from about 3.0 to about 6.5, measured at 25°C under atmospheric conditions.

In an embodiment, the method described herein additionally comprises the step (iii) removing the cosmetic composition from the keratin fibers by rinsing with water or by washing the keratin fibers with a shampoo.

### DETAILED DESCRIPTION

Embodiments of the present disclosure are discussed in detail below. In describing embodiments, specific terminology is employed for the sake of clarity. However, the disclosure is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the disclosure. While a number of embodiments and features are described herein, it is to be understood that the various features of the disclosure and aspects of embodiments, even if described separately, may be combined unless mutually exclusive or contrary to the specific description. All references cited herein are incorporated by reference as if each had been individually incorporated.

The terms "about" or "approximately," as used herein, shall generally mean within 10 percent of a given value. Other than in the operating examples, or where otherwise indicated, all numbers expressing quantities of ingredients and/or reaction conditions are to be understood as being modified in all instances by the term "about".

The ranges defined in the specification include the end values as well, i.e. a range of 1 to 10 implies that both 1 and 10 are included in the range. For the avoidance of doubt, the applicant shall be entitled to any equivalents according to applicable law.

The term "keratin fibers," as used herein, refers to a family of proteins that occur in the vertebrates and exhibit a protective function. In an embodiment, keratin fibers refer to human keratin fibers. In another embodiment, keratin fibers refer to human hair, such as human hair that grows on the scalp.

The singular form "a," "an" and "the," as used herein, include plural references unless the context clearly dictates otherwise. For example, the term "a peptide" includes a plurality of peptides, including mixtures thereof.

The term "comprising" refers to optional compatible components/steps that can be used provided that the important ingredients/steps are present.

The term "comprising" thus encompasses and includes the more restrictive terms "consisting of" and "consisting essentially of".

The term "cosmetic product" means any substance or mixture intended to be placed in contact with the external parts of the human body or with the teeth and the mucous membranes of the oral cavity with a view exclusively or mainly to cleaning them, perfuming them, changing their appearance, protecting them, keeping them in good condition or correcting body odors.

The term "cosmetic composition" (or cosmetic formulation) means any cosmetic product or preparation of the type described in Annex I ("Illustrative list by category of cosmetic products") of the Council Directive of the European Communities No. 76/768/EEC of July 27, 1976, known as the Cosmetics Directive, replaced by Regulation (EC) No 1223/2009 of the European Parliament and of the Council of 30 November 2009 on cosmetic products.

The cosmetic compositions can be formulated in a large number of types of products for the skin and/or the hair such as mousses, gels (in particular styling gels), masks for the face or the hair, conditioners, formulations for improving hairstyling, or for facilitating the combing or disentangling of the hair, for providing volume or sheen, rinsing formulations, compositions for dying or coloring the hair, hand and body lotions and oils, products for improving the moisturization of the skin, cleansing milks, make-up-removing compositions, creams or lotions for protecting against the sun and ultraviolet radiation, care and/or treatment milks and creams, anti-acne preparations, local analgesics, mascaras, products intended to be applied to the lips or other mucous membranes, sticks, deodorant and antiperspirant products, shaving lotions, bath oils and other compositions of the same type.

The term "hair product" means a cosmetic product which is intended to be applied on the hair of head or face, except eye lashes.

The term "INCI" is the acronym of International Nomenclature Cosmetic Ingredient. INCI names are systematic names internationally recognized to identify cosmetic ingredients. They are developed by the International Nomenclature Committee (INC) and published by the Personal Care Products Council (PCPC) in the International Cosmetic Ingredient Dictionary and Handbook*.*

The term "CAS RN" is the acronym of CAS Registry Number, which is a unique identification number, assigned by the Chemical Abstracts Service (CAS) to every chemical substance described in the open scientific literature, in order to index the substance in the CAS Registry, which is a collection of disclosed chemical substance information.

The cosmetic composition for use in step (i) of the method described herein comprises, in a cosmetically acceptable medium:
(a) a mixture of Hydroxypropylgluconamide and Hydroxypropylammonium Gluconate; and
(b) lactic acid, or any cosmetically acceptable salt thereof.

### Cosmetically acceptable medium

The cosmetically acceptable medium in the cosmetic composition for use in step (i) of the method described herein may be an aqueous medium including water and may include cosmetically acceptable organic solvents including alcohols such as ethyl alcohol, isopropyl alcohol, benzyl alcohol and phenylethyl alcohol, glycols or glycol ethers such as, for example, monomethyl, monoethyl and monobutyl ethers of ethylene glycol, propylene glycol or its ethers such as, for example, monomethyl ether of propylene glycol, butylene glycol, pentylene glycol, dipropylene glycol as well as the alkyl ethers of diethylene glycol such as, for example, monoethyl ether or monobutyl ether of diethylene glycol.

Water may be present in the range of about 30 to about 99 wt. %, or about 40 to about 95 wt. %, or about 45 to about 90 wt.%, based on the total weight of the composition. One or more organic solvents may be present in concentrations of between about 0.5 to about 20 wt. %, or about 2 to about 15 wt.%, based on the total weight of the cosmetic composition.

### The mixture of Hydroxypropylgluconamide and Hydroxypropylammonium Gluconate

In the cosmetic composition for use in step (i) of the method described herein, component (a) comprises a mixture of Hydroxypropylgluconamide and Hydroxypropylammonium Gluconate.

Hydroxypropylgluconamide (INCI name; CAS RN 190445-18-2) is the product obtained by the reaction of Gluconolactone with 3-amino-1-propanol, having the empirical formula C9 H19 N O7 and the structural formula

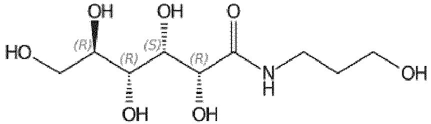

Hydroxypropylammonium Gluconate (INCI Name) is the salt of Gluconic Acid and 3-aminopropanol, having the structural formula

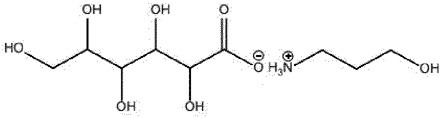

In one embodiment, in the cosmetic composition for use in step (i) of the method described herein, the molar ratio of Hydroxypropylgluconamide to Hydroxypropylammonium Gluconate in component (a) ranges from about 20:80 to about 80:20.

In one embodiment, in the cosmetic composition for use in step (i) of the method described herein, the molar ratio of Hydroxypropylgluconamide to Hydroxypropylammonium Gluconate in component (a) ranges from about 40:60 to about 60:40.

The mixture Hydroxypropylgluconamide and Hydroxypropylammonium Gluconate is commercially available from Ashland Inc. under the tradename FiberHance bm solution.

In the cosmetic composition for use in step (i) of the method described herein, the total amount of the mixture of Hydroxypropylgluconamide and Hydroxypropylammonium Gluconate (a), may be in the range of about 0.05 to about 10 wt.%, or about 0.1 to about 7.5 wt.%, or about 0.2 to about 7.5 wt.%, or about 0.3 to about 5 wt.%, or about 0.4 to about 3 wt.%, based on the total weight of the cosmetic composition.

### Lactic acid, or any cosmetically acceptable salt thereof

In the cosmetic composition for use in step (i) of the method described herein, component (b) comprises Lactic acid, or any cosmetically acceptable salt thereof.

Lactic acid (INCI Name, CAS RN 50-21-5) is a practically odorless, colorless or slightly yellow colored, viscous, hygroscopic, nonvolatile liquid. Lactic acid has the empirical formula C3 H6 O3 and the structural formula

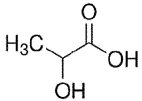

Lactic acid is chiral, consisting of two enantiomers. One is known as L-lactic acid, (S)-lactic acid, or (+)-lactic acid, and the other, its mirror image, is D-lactic acid, (R)-lactic acid, or (-)-lactic acid. It is usually in the form of the racemate, i.e. the mixture of the two enantiomers in equal amounts, called DL-lactic acid, or racemic lactic acid.

At present, lactic acid is commercially available in the form of aqueous solutions containing about 10% to about 90% by weight of lactic acid in the form of the D-and L-compounds or a racemic modification. These commercially available aqueous solutions may conveniently be used in the present disclosure. Also, it is known that L-lactic acid can be obtained by extracting fermentation products of specific plants.

In one embodiment, in the cosmetic composition for use in step (i) of the method described herein, component (b) comprises from about 60% to about 100% of L-Lactic acid (L-(+) Lactic acid; CAS RN 79-33-4), with respect to the total lactic acid content, or any cosmetically acceptable salt thereof.

In one embodiment, in the cosmetic composition for use in step (i) of the method described herein, component (b) comprises from about 90 to about 100% of L-Lactic acid, with respect to the total lactic acid content, or any cosmetically acceptable salt thereof.

The stereochemical purity of lactic acid can be determined by High-Performance Liquid Chromatography or HPLC (by separation using a chiral phase of the two enantiomers of lactic acid) or by a stereo-specific enzymatic method.

The salts of the lactic acid which can be used in the present disclosure are not particularly restricted. Suitable salts of lactic acid which can be used are alkali metal lactates, alkaline earth metal lactates, thiolactates and ammonium lactates and/or amino acid salts of lactic acid. Salts which are especially suitable are sodium lactate, calcium lactate, and potassium lactate.

Examples of the inorganic bases include hydroxides, oxides, carbonates and hydrogencarbonates of alkali metals and alkaline earth metals such as sodium, potassium, lithium, calcium and magnesium. Also, ammonium hydroxide and trialkylammonium hydroxides are useful as the inorganic bases. Examples of the organic bases include nitrogen-containing bases such as primary, secondary and tertiary amines; compounds having imino, guanidino, imidazolino, imidazolyl or pyridyl group; amino acids, peptides and proteins.

Examples of the amines as described above include those having alkyl and/or aryl groups. It is preferable to use therefore mono-, di-or trialkylamines such as propylamine, dipropylamine and triethylamine; arylamines such as aniline, methylaniline and propylaniline; monoalkanolamines, dialkanolamines, trialkanolamines and alkylalkanolamines. The amino acids are typified by glycine, alanine, valine, leucine, isoleucine, serine, threonine, cysteine, cystine, tryptophan, ornithine, carnosine (alanylhistidine), 4-aminobutanoic acid and citrulline (a-amino-a-ureidovaleric acid). Among these, the most suitable ones are basic amino acids such as lysine, histidine and arginine. Moreover, it is also possible to use, as the base, lecithin, phosphatidylethanolamine, phosphatidylserine or sphingomyelin.

In the cosmetic composition for use in step (i) of the method described herein, the total amount of lactic acid (b), or any cosmetically acceptable salt thereof, may be in the range of about 0.2 to about 10 wt.%, or about 0.3 to about 7.5 wt.%, or about 0.5 to about 7.5 wt.%, or about 0.5 to about 6 wt.%, or about 0.75 to about 5 wt.% based on the total weight of the composition.

### Cosmetic compositions

In another embodiment, the cosmetic composition for use in step (i) of the method described herein is a cosmetic composition for treating human hair.

The cosmetic composition for use in step (i) of the method described herein may comprise, in a cosmetically acceptable medium:
(a) a mixture of Hydroxypropylgluconamide and Hydroxypropylammonium Gluconate; and
(b) lactic acid, or any cosmetically acceptable salt thereof.

In the cosmetic composition for use in step (i) of the method described herein, the total amount of the mixture of Hydroxypropylgluconamide and Hydroxypropylammonium Gluconate (a), may be in the range of about 0.05 to about 10 wt.%, or about 0.1 to about 7.5 wt.%, or about 0.2 to about 7.5 wt.%, or about 0.3 to about 5 wt.%, or about 0.4 to about 3 wt.%, based on the total weight of the cosmetic composition.

In the cosmetic composition for use in step (i) of the method described herein, the total amount of lactic acid, or any cosmetically acceptable salt thereof (b), may be in the range of about 0.2 to about 10 wt.%, or about 0.3 to about 7.5 wt.%, or about 0.5 to about 7.5 wt.%, or about 0.5 to about 6 wt.%, or about 0.75 to about 5 wt.% based on the total weight of the composition.

In an embodiment, the cosmetic composition for use in step (i) of the method described herein comprises, in a cosmetically acceptable medium, based on the total weight of the composition:
(a) about 0.05-10 wt.% of a mixture of Hydroxypropylgluconamide and Hydroxypropylammonium Gluconate; and
(b) about 0.2-10 wt.% of lactic acid, or any cosmetically acceptable salt thereof.

In an embodiment, the cosmetic composition for use in step (i) of the method described herein comprises, in a cosmetically acceptable medium, based on the total weight of the composition:
(a) about 0.1-5 wt.% of a mixture of Hydroxypropylgluconamide and Hydroxypropylammonium Gluconate; and
(b) about 0.3-7.5 wt.% of lactic acid, or any cosmetically acceptable salt thereof.

In an embodiment, the cosmetic composition for use in step (i) of the method described herein comprises, in a cosmetically acceptable medium, based on the total weight of the composition:
(a) about 0.2-7.5 wt.% of a mixture of Hydroxypropylgluconamide and Hydroxypropylammonium Gluconate, wherein the molar ratio of Hydroxypropylgluconamide to Hydroxypropylammonium Gluconate in component (a) ranges from about 20:80 to about 80:20; and
(b) about 0.5-7.5 wt.% of L-lactic acid, or any cosmetically acceptable salt thereof.

In an embodiment, the cosmetic composition for use in step (i) of the method described herein comprises, in a cosmetically acceptable medium, based on the total weight of the composition:
(a) about 0.3-5 wt.% of a mixture of Hydroxypropylgluconamide and Hydroxypropylammonium Gluconate, wherein the molar ratio of Hydroxypropylgluconamide to Hydroxypropylammonium Gluconate in component (a) ranges from about 20:80 to about 80:20; and
(b) about 0.5-6 wt.% of L-lactic acid, or any cosmetically acceptable salt thereof.

In an embodiment, the cosmetic composition for use in step (i) of the method described herein comprises, in a cosmetically acceptable medium, based on the total weight of the composition:
(a) about 0.4-3 wt.% of a mixture of Hydroxypropylgluconamide and Hydroxypropylammonium Gluconate, wherein the molar ratio of Hydroxypropylgluconamide to Hydroxypropylammonium Gluconate in component (a) ranges from about 20:80 to about 80:20; and
(b) about 0.75-5 wt.% of L-lactic acid, or any cosmetically acceptable salt thereof.

In an embodiment, the cosmetic composition for use in step (i) of the method described herein comprises, in a cosmetically acceptable medium, based on the total weight of the composition:
(a) about 0.4-3 wt.% of a mixture of Hydroxypropylgluconamide and Hydroxypropylammonium Gluconate, wherein the molar ratio of Hydroxypropylgluconamide to Hydroxypropylammonium Gluconate in component (a) ranges from about 40:60 to about 60:40; and
(b) about 0.75-5 wt.% of L-lactic acid, or any cosmetically acceptable salt thereof.

In an embodiment, in the cosmetic composition for use in step (i) of the method described herein, the weight ratio between (a) the mixture of Hydroxypropylgluconamide and Hydroxypropylammonium Gluconate and (b) lactic acid, or any cosmetically acceptable salt thereof, ranges from about 0.1:1 to about 50:1, or from about 0.2:1 to about 30:1, or from about 0.3:1 to about 10:1, or from about 0.3:1 to about 5:1.

### Other Components

The cosmetic composition for use in step (i) of the method described herein may contain a variety of other components to enhance the beneficial properties of this composition, as further described herein.

### 1. Thickening Agents

In an embodiment, the compositions of the present disclosure may contain one or more agents that will provide a thickening, or viscosity increasing effect, to the compositions. Suitable thickening agents include, but are not limited to, anionic, synthetic polymers, cationic, synthetic polymers, naturally occurring thickeners, such as nonionic guar gums, scleroglucan gums or xanthan gums, gum arabic, gum ghatti, karaya gum, tragacanth gum, carrageenan gum, agar-agar, locust bean gum, pectins, alginates, starch fractions, and derivatives such as amylose, amylopectin, and dextrins, as well as cellulose derivatives such as, for example, methylcellulose, carboxyalkylcelluloses, and hydroxyalkylcelluloses (such as hydroxyethylcellulose, hydroxyethyl ethylcellulose, hydroxypropylcellulose or hydroxypropyl methylcellulose), nonionic, fully synthetic polymers, such as polyvinyl alcohol or polyvinylpyrrolidinone; as well as inorganic thickeners, such as phyllosilicates, for example, bentonite, or smectites, such as montmorillonite or hectorite. Thickening agents may be included at from about 0.001-20 wt.%, or about 0.005-15 wt.%, or about 0.01-12 wt.%, based on the total weight of the composition.

### 2. Preservatives

"Preservatives", as used herein, are ingredients which prevent or retard microbial growth and thus help protect cosmetic products from spoilage. Preservatives may also protect the product from inadvertent contamination by the consumer during use. Suitable preservatives include, but are not limited to, methyl, ethyl, and propyl paraben, hydantoins, phenoxyethanol, dehydroacetic acid, and the like. Preservatives may be included at about 0.0001-8 wt.%, or about 0.0005-7 wt.%, or about 0.001-5 wt.%, based on the total weight of the composition.

### 3. Chelating Agents

The compositions of the present disclosure may contain about 0.0001-5 wt.%, or 0.0005-3 wt.%, or 0.001-2 wt.%, based on the total weight of the composition, of one or more chelating agents. Chelating agents may be capable of complexing with and inactivating metallic ions in order to help prevent their adverse effects on the stability or effects of the composition. Suitable chelating agents include, but are not limited to, sodium citrate; nitrogen-containing polycarboxylic acids, such as Ethylenediaminetetraacetate (EDTA), and calcium, sodium, potassium or triethanolamine derivatives thereof, Hydroxyethyl Ethylenediamine Triacetic Acid (HEDTA), Ethylenediamine-N,N'-disuccinic acid (EDDS); and phosphonates, such as 1-hydroxyethane-1,1-diphosphonate (HEDP), and/or ethylenediamine tetramethylene phosphonate (EDTMP), and/or diethylenetriamine pentamethylene phosphonate (DTPMP), or sodium salts thereof.

### 4. pH Adjusters

In embodiments, acids, bases or buffering systems may be added to adjust the pH of the compositions of the present disclosure to the desired pH range. Suitable acids include hydrochloric acid, phosphoric acid, citric acid, and the like. Suitable bases include sodium or potassium hydroxide, monoethanolamine, diethanolamine, triethanolamine, aminomethyl propanol, and the like. pH adjusters may be included at from about 0.00001-8 wt.%, or about 0.00005-6 wt.%, or about 0 0001-5 wt.%, based on the total weight of the composition.

### 5. Pigments

The compositions of the present disclosure may contain inorganic pigments. Suitable inorganic pigments include, but are not limited to, those having a color index number as listed in the CTFA dictionary, 10th edition, 2004, hereby incorporated by reference.

### 6. Direct dyes

The compositions of the present disclosure may contain direct dyes. Direct dye, also called substantive dye, is a dye that adheres to its substrate by non-ionic forces, e.g., without help from other chemicals. For purposes of this disclosure dyes include direct dyes selected from anionic, cationic and non-ionic nitro dyes.

Examples of anionic direct dyes include, but are not limited to, Bromophenol Blue, Tetrabromophenol Blue, Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 5, FD&C Yellow No. 6, FD&C Blue 1, Food Black 1, Food Black 2, Disperse Black 9 and Disperse Violet 1, and their alkali metal salts such as sodium or potassium.

Examples of cationic dyes include, but are not limited to, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12, Basic Red 22, Basic Red 76, Basic Violet 1, Basic Violet 2, Basic Violet 3, Basic Violet 10, Basic Violet 14, Basic Yellow 57, Basic red 51, Basic Yellow 87 and Basic Orange 31.

Examples of non-ionic nitro dyes (HC dyes) include, but are not limited to, HC Blue No. 2, HC Blue No. 4, HC Blue No. 5, HC Blue No. 6, HC Blue No. 7, HC Blue No. 8, HC Blue No. 9, HC Blue No. 10, HC Blue No. 11, HC Blue No. 12, HC Blue No. 13, HC Blue No. 15, Blue No. 16, HC Blue No. 18, HC Brown No. 1, HC Brown No. 2, HC Green No. 1, HC Orange No. 1, HC Orange No. 2, HC Orange No. 3, HC Orange No. 5, HC Red BN, HC Red No. 1, HC Red No. 3, HC Red No. 7, HC Red No. 8, HC Red No. 9, HC Red No. 10, HC Red No. 11, HC Red No. 13, HC Red No. 14, HC Red No. 18, HC Red No. 54, HC Violet BS, HC Violet No. 1, HC Violet No. 2, HC Yellow No. 2, HC Yellow No. 4, HC Yellow No. 5, HC Yellow No. 6, HC Yellow No. 7, HC Yellow No. 8, HC Yellow No. 9, HC Yellow No. 10, HC Yellow No. 11, HC Yellow No. 12, HC Yellow No. 13, HC Yellow No. 14, HC Yellow No. 15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

### 7. Fragrance or perfumes

The compositions of the present disclosure may include one or more fragrances or perfumes. For purposes of this disclosure, the term "fragrance" refers to any substance, natural or synthetic, used to impart an odor to a product.

For purposes of this disclosure, the term "perfume" refers to any mixture of fragrant essential oils and aroma compounds, fixatives, and solvents used to give the human body, objects, and living spaces a lasting and pleasant smell.

### 8. Anionic surfactants

The compositions of the present disclosure may include one or more anionic surfactants. Non-limiting examples of anionic surfactants include, but are not limited to, alkyl ether carboxylates, alkyl sulfates, alkyl ether sulfates, amide ether sulfates, alkyl glyceride sulfates, olefin sulfonates, alkyl-aryl sulfonates, sulfosuccinates, sulfo fatty acid esters, fatty acid isethionates, fatty acid taurides, phosphate esters, acyl glutamates, acyl peptides, acyl sarcosides, and mixtures thereof.

Examples of alkyl ether carboxylates, alkyl sulfates, alkyl ether sulfates, olefin sulfonates, and mixtures thereof include Potassium Laureth-4 Carboxylate, Sodium Laureth-4 Carboxylate, Sodium Laureth-5 Carboxylate, Sodium Laureth-6 Carboxylate, and Sodium Laureth-11 Carboxylate, Ammonium Laureth Sulfate, Monoethanolamine (MEA)-Laureth Sulfate, Monoisopropanolamine (MIPA)-Laureth Sulfate, Sodium Laureth Sulfate, and Triethanolamine (TEA)-Laureth Sulfate, Sodium C14-C16 Olefin Sulfonate, and mixtures thereof.

### 9. Cationic surfactants

The compositions of the present disclosure may include one or more cationic surfactants. Non-limiting examples of cationic surfactants include, but are not limited to, alkylimidazolines, tetra alkyl(-aryl) quaternary ammonium salts (quats), heterocyclic ammonium salts, quaternized alkyl polyglycosides, quaternized derivatives of polyalkanolamine esters (esterquats), and mixtures thereof.

Examples of C6-C24 alkyl trimethyl quaternary ammonium salts, C6-C24 alkyl dimethyl benzyl quaternary ammonium salts, C6-C24 dialkyl dimethyl quaternary ammonium salts, and mixtures thereof include Laurtrimonium bromide, Laurtrimonium chloride, Myrtrimonium bromide, Myrtrimonium chloride, Cetrimonium bromide, Cetrimonium chloride, Cetrimonium methosulfate, Steartrimonium bromide, Steartrimonium chloride, Steartrimonium methosulfate, Behentrimonium bromide, Behentrimonium chloride, Behentrimonium methosulfate, Ceteartrimonium chloride, Cocotrimonium chloride, Cocotrimonium methosulfate, Soytrimonium chloride, Octyl dodecyl trimethyl ammonium chloride, Dodecyl hexadecyl trimethyl ammonium bromide, Dodecyl hexadecyl trimethyl ammonium chloride, Benzalkonium chloride, benzyl-C10-16-alkyldimethylammonium chloride, benzyl-C12-14-alkyldimethylammonium chloride, Didecyldimonium chloride, Dilauryldimonium chloride, Distearyldimonium chloride, Behenoyl PG-Trimonium Chloride, Dioleoylethyl Hydroxyethylmonium Methosulfate, and mixtures thereof.

### 10. Nonionic surfactants

The compositions of the present disclosure may include one or more nonionic surfactants. Non-limiting examples of nonionic surfactants include, but are not limited to, polyethoxylated and/or polypropoxylated C6-C24 fatty alcohols, polyethoxylated and/or polypropoxylated fatty acids, alkylphenols, alpha-diols or alcohols having a fatty chain containing, for example, 8 to 18 carbon atoms, ethylene oxide or propylene oxide groups to range from 2 to 50; copolymers of ethylene oxide and of propylene oxide, polyethoxylated fatty amides having from 2 to 30 mol of ethylene oxide; polyglycerolated fatty amides containing on average 1 to 5, or 1.5 to 4, glycerol groups; polyethoxylated fatty amines having 2 to 30 mol of ethylene oxide; oxyethylenated fatty acid esters of sorbitan having from 2 to 30 mol of ethylene oxide; polyethoxylated castor oil, polyethoxylated hydrogenated castor oil, fatty acid esters of sucrose; fatty acid esters of polyethylene glycol; alkylpolyglycosides; N-alkylglucamine derivatives; amine oxides such as (C10-C14) alkylamine oxides or N-acylaminopropylmorpholine oxides.

### 11. Amphoteric or zwitterionic surfactants

The compositions of the present disclosure may include one or more amphoteric or zwitterionic surfactants. Non-limiting examples of amphoteric or zwitterionic surfactants include, but are not limited to, aliphatic secondary or tertiary amine derivatives in which the aliphatic radical is a linear or branched chain containing 8 to 18 carbon atoms and containing at least one water-soluble anionic group (for example carboxylate, sulphonate, sulphate, phosphate or phosphonate); (C8-C20) alkylbetaines, sulphobetaines, (C8-C20) alkylamido (C1-C6) alkylbetaines or (C8-C20) alkylamido (C1-C6) alkylsulphobetaines; amine derivatives, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, and cocoamphodipropionic acid.

### 12. Petroleum hydrocarbons (mineral oils, paraffins and waxes)

The compositions of the present disclosure may include one or more petroleum hydrocarbons. For purposes of this disclosure petroleum hydrocarbons refer to mineral oils, paraffins and waxes based on petroleum. Examples include, but are not limited to, hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures that consist mainly of saturated C18-C30 hydrocarbons and smaller amounts of iso-alkanes and cycloalkanes with a molecular weight between 250 and 450 g/mol and, although they are solids at room temperature, they have low melting points, usually between 40°C and 60°C), microcrystalline paraffin waxes (which consist of C40-C55 compounds that contain, in addition to normal hydrocarbons, included in the group are iso-alkanes and naphtenes with long alkyl side-chains, the iso-alkanes forming microcrystals, the microcrystalline paraffin waxes having mean molecular weights between 500 and 800 g/mol, being solids at room temperature, and having melting points between 60°C and 90°C), or mixtures thereof.

### 13. C6-C24 fatty alcohols

The compositions of the present disclosure may include one or more C6-C24 fatty alcohols. C6-C24 fatty alcohols from vegetable fats and oils include cotton, safflower, coconut, rapeseed, linseed, palm, palm kernel, sunflower, olein, olive, olive pomace, castor oil, soy, tall oil, etc., totally or partially hydrogenated, as well as purified or synthetic fatty alcohols such as caproyl alcohol, capryl alcohol, capric alcohol, lauryl alcohol, myristyl alcohol, palmytil (cetyl) alcohol, palmitoyl alcohol, stearyl alcohol, isostearyl alcohol, 2-octyldodecanol, 2-ethylhexanoyl alcohol, oleyl alcohol, ricinoleyl alcohol, elaidyl alcohol, petroselinic alcohol, linoleyl alcohol, linolenyl alcohol, arachidyl alcohol, gadoleyl alcohol, behenyl alcohol and erucyl alcohol, or technical grade mixtures.

### 14. Vegetable fats and oils

The compositions of the present disclosure may include one or more vegetable fats and/or oils. For purposes of this disclosure, vegetable fats and oils are linear and/or branched esters, linear or branched, saturated and/or unsaturated alkanecarboxylic acids with a chain length of 1 up to 30 carbon atoms, saturated and/or unsaturated alcohols with a chain length of 1 up to 30 carbon atoms; or linear and/or branched esters of aromatic carboxylic acids, or saturated and/or unsaturated alcohols with a chain length of 1 up to 30 carbon atoms. These oils can be selected from, for example, isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl laurate, 2-ethylhexyl palmitate, 2-ethylhexyl cocoate, 2-hexyldecyl stearate, 2- ethylhexyl isostearate, 2-octyldodecyl palmitate, cetyl palmitate, stearyl palmitate, oleyl palmitate, oleyl oleate, oleyl erucate, erucyl oleate, erucyl erucate, as well as synthetic, semisynthetic and natural mixtures esters, such as jojoba oil (a natural mixture of esters of monounsaturated monocarboxylic acids with a C18-C24 chain with also monounsaturated monoalcohols and with a long C18-C24 chain).

Other examples of vegetable fats and oils include ester oils such as sugar esters or diesters of C12-C24 fatty acids. The term "sugar" means compounds comprising several alcohol functional groups, with or without an aldehyde or ketone function, and which comprise at least 4 carbon atoms. These sugars may be monosaccharides, oligosaccharides or polysaccharides.

Non-limiting examples of sugars that may be used according to the present disclosure include sucrose (or saccharose), glucose, galactose, ribose, fucose, maltose, fructose, mannose, arabinose, xylose and lactose, and derivatives thereof, including for example, alkyl derivatives such as methyl derivatives, for instance methylglucose. Non-limiting examples of the sugar esters of fatty acids that may be used according to this disclosure include those from the group comprising esters or mixtures of esters of sugars described above and of linear or branched, saturated or unsaturated C12-C24 fatty acids.

The esters may be chosen from mono-, di-, tri-, tetraesters and polyesters, and mixtures thereof. These esters may be chosen from, for example, oleates, laurates, palmitates, myristates, behenates, cocoates, stearates, linoleates, linolenates, caprates and arachidonates, or mixtures thereof such as oleo-palmitate, oleo-stearate and palmito-stearate mixed esters. The sucrose, glucose or methylglucose monoesters and diesters and for example sucrose, glucose or methylglucose mono- or dioleates, stearates, behenates, oleopalmitates, linoleates, linolenates and oleostearates, constitute sugar esters or diesters of C12-C24 fatty acids that are suitable in the context of the present disclosure. One example is methylglucose dioleate.

Other suitable oils of the type of esters of saturated alkane carboxylic acids and alcohols are fatty acid methyl esters, such as C12-C24 fatty acid methyl esters from animal and vegetable fats and oils such as cotton, safflower, coconut, rapeseed, linseed, palm, palm kernel, sunflower, olein, olive, olive pomace, castor oil, soy, tall oil, etc., totally or partially hydrogenated, as well as purified or synthetic fatty acids such as caproic acid, caprylic acid, capric acid, lauric acid, myristic acid, palmitic acid (cetylic acid), palmitoleic acid, stearic acid, isostearic acid, 2-ethylhexanoic acid, oleic acid, ricinoleic acid, elaidic acid, petroselinic acid, linoleic acid, linolenic acid, arachidic acid, gadoleic acid, behenic acid and erucic acid, or mixtures thereof.

Other suitable vegetable fats and oils according to the present disclosure are fatty acid triglycerides, including for example, linear and/or branched triglycerin esters, saturated and/or unsaturated alkanecarboxylic acids with a chain length of 6 up to 24 carbon atoms, such as 10 up to 18 carbon atoms. The fatty acids esterifying the different positions of glycerin can be different, giving rise to a large amount of possible combinations, including positional combinations. The position of the different fatty acids in natural triglycerides is not random, but rather it depends on the origin of the fat. The most simple triglycerides are those constituted by a sole fatty acid.

Fatty acid triglycerides can be chosen, for example, from synthetic, semi-synthetic and natural oils, for example avocado oil, almond oil, hazelnut oil, babassu palm oil, borage oil, peanut oil, canola oil, hemp oil, milk thistle oil, safflower oil, chufa oil, coconut oil, rapeseed oil, black cumin oil, wheat germ oil, sunflower oil, linseed oil, macadamia nut oil, corn oil, walnut oil, olive oil and its by-products such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like.

### 15. Natural waxes

The compositions of the present disclosure may include one or more natural waxes. Natural waxes according to the present disclosure, include but are not limited to, candelilla wax, carnauba wax, Japan wax, esparto wax, cork wax, guaruma wax, rice wax, sugar cane wax, ouricury wax, montan wax, beeswax, shellac wax, espermaceti, wool lanolin (wax), uropygial fat wax, ceresin waxes, peat waxes, ozokerite, as well as chemically modified waxes (hard waxes) for example, montan wax esters, waxes obtained by the Fischer-Tropsch process, hydrogenated jojoba waxes and synthetic waxes.

### 16. Silicones

Non-limiting examples of silicones include, but are not limited to, cyclic and/or linear silicones, which can be found as monomers generally characterized by structural elements such as: 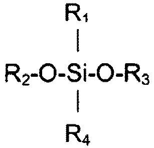 where the silicon atoms can be substituted by alkyl or aryl radicals the same or different, represented here generally by R₁-R₄ groups.

Linear silicones with siloxane units suitable according to the present disclosure are generally characterized by structural elements such as: 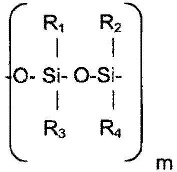 where the silicon atoms can be substituted by alkyl or aryl radicals the same or different, represented here in general by R₁-R₄ groups (meaning the number of different radicals is not limited to 4), and m can range from 2 to 200.000.

Cyclic silicones suitable according to the present disclosure are generally characterized by structural elements such as: 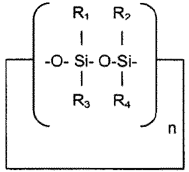 where the silicon atoms can be substituted by alkyl or aryl radicals the same or different, represented here generally by R₁-R₄ groups (meaning the number of different radicals is not limited to 4), and n can range from 3/2 to 20. Fractional values of n indicate that odd numbers of siloxane groups may be present in the ring.

Examples include a cyclic methyl siloxane having the formula [(CH₃)₂SiO]x in which x is 3-6, or short chain linear methyl siloxanes having the formula ((CH₃)₂SiO[(CH₃)₂SiO]_{y}Si(CH₃)₃ in which y is 0-5.

Some suitable cyclic methyl siloxanes are hexamethylcyclotrisiloxanes (D3), octamethylcyclotetrasiloxane (D4), decamethylcyclopentasiloxane (D5) (cyclomethicone) and dodecamethylcyclohexasiloxane (D6).

Some suitable short linear methyl siloxane are hexamethyldisiloxane (MM), octamethyltrisiloxane (MDM), decamethyltetrasiloxane (MD2M), dodecamethylpentasiloxane (MD3M), tetradecamethylhexasiloxane (MD4M), and hexadecamethylheptasiloxane (MD5M).

Long chain linear siloxanes such as phenyltrimethicone, bis(phenylpropyl)dimethicone, dimethicone, dimethiconol, cetyldimethicone and behenoxy dimethicone are also suitable silicones according to the present disclosure.

### 17. Cationic Polymers

The compositions of the present disclosure may include one or more cationic polymers. The term "cationic polymer" as used herein refers to a macromolecule (polymer) that contains at least one monomer bearing a quaternary ammonium group.

Examples of suitable cationic polymers include, but are not limited to, those polymers known by their International Nomenclature for Cosmetic Ingredients (INCI) name as Polyquaternium. Some examples of those are Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-42, Polyquaternium-43, Polyquaternium-44, Polyquaternium-45, Polyquaternium-46, Polyquaternium-47, Polyquaternium-48, Polyquaternium-49, Polyquaternium-50, Polyquaternium-51, Polyquaternium-52, Polyquaternium-53, Polyquaternium-54, Polyquaternium-55, Polyquaternium-56, Polyquaternium-57, Polyquaternium-58, Polyquaternium-59, Polyquaternium-60, Polyquaternium-61, Polyquaternium-62, Polyquaternium-63, Polyquaternium-64, Polyquaternium-65, Polyquaternium-66, Polyquaternium-67, Polyquaternium-68, Polyquaternium-69, Polyquaternium-70, Polyquaternium-71, Polyquaternium-72, Polyquaternium-73, Polyquaternium-74, Polyquaternium-75, Polyquaternium-76, Polyquaternium-77, Polyquaternium-78, Polyquaternium-79, Polyquaternium-80, Polyquaternium-81, Polyquaternium-82, Polyquaternium-83, Polyquaternium-84, Polyquaternium-85, Polyquaternium-86, Polyquaternium-87, Polyquaternium-88, Polyquaternium-91, Polyquaternium-92, Polyquaternium-94, Polyquaternium-95, Polyquaternium-96, Polyquaternium-98, Polyquaternium-99, Polyquaternium-100, Polyquaternium-102, Polyquaternium-104, Polyquaternium-109, Polyquaternium-110, Polyquaternium-111, Polyquaternium-112, Polyquaternium-113 and Polyquaternium-114. Other suitable cationic polymers include those known by their International Nomenclature for Cosmetic Ingredients (INCI) name as Guar hydroxypropyl trimonium chloride, Polyacrylamidopropyltrimonium Chloride, Polymethacrylamidopropyltrimonium Chloride and Polymethacrylamidopropyltrimonium Methosulfate.

### 18. Non-ionic polymers

The compositions of the present disclosure may include one or more non-ionic polymers. Non-limiting examples of non-ionic polymers include, but are not limited to: nonionic guar gums and modified nonionic guar gums, which may be those modified with C1-C6 hydroxyalkyl groups; biopolysaccharide gums of microbial origin such as scleroglucan gum or xanthan gum; gums derived from plant exudates, such as gum arabic, ghatti gum, karaya gum, tragacanth gum, carrageenan, agar and carob gum; pectins; alginates; starches; hydroxy (C1-C6) alkylcelluloses and carboxy (C1-C6) alkylcelluloses; celluloses modified with groups comprising at least one fatty chain; non-limiting examples include hydroxyethylcelluloses modified with groups comprising at least one fatty chain, such as alkyl, arylalkyl or alkylaryl groups, or mixtures thereof, and wherein the alkyl groups are, for example, C8-C22; or those modified with polyalkylene glycol alkylphenyl ether groups; hydroxypropyl guars modified with groups comprising at least one C8-C22 fatty chain, copolymers of vinylpyrrolidone and of hydrophobic monomers comprising a fatty chain, such as vinylpyrrolidone/hexadecene copolymer or vinylpyrrolidone/eicosene copolymer; copolymers of C1-C6 alkyl acrylates or methacrylates and of amphiphilic monomers comprising at least one fatty chain; copolymers of hydrophilic acrylates or methacrylates and of hydrophobic monomers comprising at least one fatty chain, such as the polyethylene glycol methacrylate/lauryl methacrylate copolymer; polymers with an aminoplast ether skeleton comprising at least one fatty chain; and polyurethane polyethers comprising in their chain both hydrophilic blocks, for example of polyoxyethylenated nature, and hydrophobic blocks that may be aliphatic blocks alone and/or cycloaliphatic and/or aromatic blocks.

### 19. Amphoteric polymers

The compositions of the present disclosure may include one or more amphoteric polymers. Non-limiting examples of amphoteric polymers include, but are not limited to, amphoteric polysaccharides such as Carboxymethylchitosan or N-[(2'-Hydroxy-2',3'-dicarboxy)ethyl]chitosan; Amphoteric Urethanes; Modified Potato Starch; Methacryloyl Ethyl Betaine/Acrylates Copolymer; Acrylates/Lauryl Acrylate/Stearyl Acrylate/Ethylamine Oxide Methacrylate; and Acrylic acid/(meth)acrylamidopropyl-trimethylamonium chloride/stearyl methacrylate terpolymer.

### 20. Anionic polymers

The compositions of the present disclosure may include one or more anionic polymers. Non-limiting examples of anionic polymers include, but are not limited to, those polymers known by their International Nomenclature for Cosmetic Ingredients (INCI) name as Shellac, Acrylates Copolymer, Acrylates/C1-2 Succinates/Hydroxyacrylates Copolymer, Acrylic Esters (and) Methacrylic Esters Copolymer, AMP-Acrylates/Allyl Methacrylate Copolymer,

Diglycol/Cyclohexanedimethanol/Isophtalates/Sulfoisophtalates Copolymer, Diglycol/Isophtalates/Sulfoisophtalates Copolymer, Isobutylene Ethylmaleimide/Hydroxyethylmaleimide Copolymer, Glycerin and Diglycol/Cyclohexanedimethanol/Isophtalates/Sulfoisophtalates Copolymer, Methacrylate Acid/Sodium Acrylamidomethyl Propane Sulfonate Copolymer, Octylacrylamide/Acrylates/Butylaminoethylmethacrylate Copolymer, Polyurethane (and) Acrylates Copolymer, PVM/MA Copolymer, PVP/Ethyl Methacrylate/Methacrylic Acid Terpolymer, PVP/Polycarbamyl Polyglycol Ester, VA/Crotonates Copolymer, VA/Crotonates/Vinyl Neodecanoate Copolymer, VP/Acrylates/Lauryl Methacrylate Copolymer, and their cosmetically acceptable salts.

### 21. Other additives

The compositions of the present disclosure may include one or more additives other than those described above. Non-limiting examples of additives different from those listed above include, but are not limited to, Vitamins and Provitamins, such as beta-carotene, retinal, retinol, retinoic acid, biotin, panthenol, panthenyl ethyl ether, ascorbic acid, ascorbyl glucoside, ascorbyl palmitate, ascorbyl phosphate, tocopherol, tocopheryl acetate, tocopheryl glucoside, tocopheryl palmitate, and the like; hair strengthening agents such as maleic acid, and the like; antioxidants (ingredients employed in cosmetics to prevent or retard product spoilage from rancidity or deterioration from reaction with oxygen); moisturizers such as lactic acid or allantoin; natural extracts (substances or active ingredients with desirable properties that are removed from a plant, flower, alga, fungus or bacteria); UV filters (ingredients used to absorb or reflect the UV rays that are contained in sun light or in artificial light); or mixtures thereof.

In an embodiment, the cosmetic composition for use in step (i) of the method described herein comprises, in a cosmetically acceptable medium,
(a) a mixture of Hydroxypropylgluconamide and Hydroxypropylammonium Gluconate;
(b) lactic acid, or any cosmetically acceptable salt thereof; and
(c) optionally, at least one additional cosmetic ingredient selected from thickening agents; preservatives; chelating agents; pH adjusters; pigments; direct dyes; fragrance or perfumes; anionic surfactants; cationic surfactants; nonionic surfactants; amphoteric or zwitterionic surfactants; petroleum hydrocarbons (mineral oils, paraffins and waxes); C6-C24 fatty alcohols; vegetable fats and oils; natural waxes; silicones; cationic polymers; non-ionic polymers; anionic polymers; and other additives such as vitamins and provitamins; hair strengthening agents; antioxidants; moisturizers; natural extracts; UV filters; or mixtures thereof.

In an embodiment, the cosmetic composition for use in step (i) of the method described herein comprises, in a cosmetically acceptable medium, based on the total weight of the composition:
a) about 0.05-10 wt.% of a mixture of Hydroxypropylgluconamide and Hydroxypropylammonium Gluconate;
b) about 0.2-10 wt.% of lactic acid, or any cosmetically acceptable salt thereof; and
c) optionally, at least one additional cosmetic ingredient selected from thickening agents; preservatives; chelating agents; pH adjusters; pigments; direct dyes; fragrance or perfumes; anionic surfactants; cationic surfactants; nonionic surfactants; amphoteric or zwitterionic surfactants; petroleum hydrocarbons (mineral oils, paraffins and waxes); C6-C24 fatty alcohols; vegetable fats and oils; natural waxes; silicones; anionic polymers; and other additives such as vitamins and provitamins; hair strengthening agents; antioxidants; moisturizers; natural extracts; UV filters; or mixtures thereof.

In an embodiment, the cosmetic composition for use in step (i) of the method described herein comprises, in a cosmetically acceptable medium:
a) a mixture of Hydroxypropylgluconamide and Hydroxypropylammonium Gluconate;
b) lactic acid, or any cosmetically acceptable salt thereof; and
c) optionally, maleic acid or any cosmetically acceptable salt thereof.

In an embodiment, the cosmetic composition for use in step (i) of the method described herein comprises, in a cosmetically acceptable medium, based on the total weight of the composition:
a) about 0.05-10 wt.% of a mixture of Hydroxypropylgluconamide and Hydroxypropylammonium Gluconate;
b) about 0.2-10 wt.% of lactic acid, or any cosmetically acceptable salt thereof; and
c) optionally, about 0.2-10 wt.% of maleic acid or any cosmetically acceptable salt thereof.

### Suitable cosmetic forms

No particular limitation is imposed on the form of the cosmetic composition for use in step (i) of the method described herein. Examples include hair sprays, setting lotions, mousses, gels, waxes, creams, pomades, gums or serums (Thomas Clausen et al., Hair Preparations, Ullmann's Encyclopedia of Industrial Chemistry, 2006).

In embodiments, the compositions are formulated as a hair spray, a setting lotion or a mousse.

Hair sprays may be aerosol sprays, which may be two-phase aerosols in which both a liquid and a gaseous phase are present inside the container; or pump sprays (non-aerosol sprays), which do not employ a propellant gas and the energy needed to swirl and atomize the liquid must be supplied by a pump, which is operated with a finger or through a lever.

Setting lotions may be thin, aqueous-alcohol solutions in single- or multiple-application packages.

Mousses may be thin, aqueous or aqueous-alcohol solutions in, for example, alumina cans with a propellant; or non-aerosol mousses, where the mousse is generated through use of a squeeze or pump dispenser with a riddle in the head of the pump. The liquid is mixed with air in the head of the pump and dispensed as mousse.

The cosmetic composition for use in step (i) of the method described herein may have a viscosity in the range of about 1 mPas to about 250,000 mPas, or in the range of about 5 mPas to about 200,000 mPas, or in the range of about 10 mPas to about 50,000 mPas, or in the range of about 100 mPas to about 25,000 mPas, or in the range of about 1,000 to about 15,000 mPas, measured with a Brookfield Dial Reading Viscometer LVT (spindle D at 12 rpm) at 25°C under atmospheric conditions.

The cosmetic composition for use in step (i) of the method described herein may have a pH in the range from about 3.0 to about 6.5, or about 3.5 to about 5.0, measured at 25°C under atmospheric conditions.

In one embodiment, the cosmetic compositions for use in step (i) of the method described are "rinse-off" compositions. The term "rinse-off" as used herein refers to compositions that contain ingredients intended to be applied and left on the hair for short periods of time (i.e. about 5 s to about 1 hour, or about 10 s to 45 min) and subsequently be removed by water and/or shampooing the hair.

### Treatment process

In an embodiment, it is described a method for increasing the elasticity of keratin fibers comprising the steps of:
(i) treating the keratin fibers by topical application of a cosmetic composition comprising, in a cosmetically acceptable medium:
   (a) a mixture of Hydroxypropylgluconamide and Hydroxypropylammonium Gluconate; and
   (b) lactic acid, or any cosmetically acceptable salt thereof;
(ii) allowing the composition to remain in contact with the keratin fibers for a period ranging from about 5 seconds to about 1 hour (60 minutes).

In one embodiment, in the method described herein, in step (ii) the cosmetic composition is left in contact with the keratin fibers for a period ranging from about 5 seconds to about 1 hour (60 minutes), or from about 10 seconds to about 45 minutes, or from about 15 seconds to about 30 minutes.

In an embodiment, in the method described herein, the keratin fibers are human hair.

In an embodiment, in the method described herein, human hair has been previously damaged as a result of at least one treatment selected from hair bleaching, hair dyeing, hair straightening, hair permanent wave (hair perm), blow-drying, or combinations thereof.

In an embodiment, in the method described herein, human hair has been previously damaged as a result of at least one treatment selected from hair bleaching, hair dyeing, or combinations thereof.

Hair bleaching refers to lightening the hair. This is usually achieved by oxidation using hydrogen peroxide as the principal oxidizing agent, and salts of persulfate added as "accelerators".

Hair dying (or hair coloring) refers to changing the color of the hair on humans' heads. This is achieved by using chemical compounds, i.e. dyes, which can be obtained from natural sources (such as henna) or are synthetic compounds.

Hair straightening refers to hair treatments that straighten hair. This can be achieved by combining chemical compounds and hair straightener (such as flat iron).

Hair permanent wave (hair perm) refers to hair treatments that permanently alter the hair texture by breaking the bonds of hair and resetting them. Typically, a perm service involves two key elements. First, the hair strands are wrapped in perm rods and apply a perm solution is applied. Then, the perm solution is rinse off, the hair is dried, and a neutralizer is applied to complete the process. Once the neutralizer solution is completely rinsed off, the new texture will be revealed.

Blow-drying refers to using a blow dryer to dry the keratin fibers for more than 15 minutes at more than three non-immediately successive instances in a 24 hour period.

In an embodiment, the method described herein additionally comprises the step (iii) removing the cosmetic composition from the keratin fibers by rinsing with water or by washing the keratin fibers with a shampoo.

In an embodiment, the method described herein additionally comprises the step (iiib) removing the cosmetic composition from the keratin fibers by washing the keratin fibers with a shampoo.

In an embodiment, the method described herein additionally comprises the step (iv) rinsing the keratin fibers with water.

In an embodiment, it is described a method for increasing the elasticity of keratin fibers comprising the steps of:
(i) treating the keratin fibers by topical application of a cosmetic composition comprising, in a cosmetically acceptable medium:
   (a) a mixture of Hydroxypropylgluconamide and Hydroxypropylammonium Gluconate; and
   (b) lactic acid, or any cosmetically acceptable salt thereof;
(ii) allowing the composition to remain in contact with the keratin fibers for a period ranging from about 5 seconds to about 1 hour; and
(iii) removing the cosmetic composition from the keratin fibers by rinsing with water or by washing the keratin fibers with a shampoo.

In an embodiment, it is described a method for increasing the elasticity of keratin fibers comprising the steps of:
(i) treating the keratin fibers by topical application of a cosmetic composition comprising, in a cosmetically acceptable medium:
   (a) a mixture of Hydroxypropylgluconamide and Hydroxypropylammonium Gluconate; and
   (b) lactic acid, or any cosmetically acceptable salt thereof;
(ii) allowing the composition to remain in contact with the keratin fibers for a period ranging from about 10 seconds to 45 minutes; and
(iii) removing the cosmetic composition from the keratin fibers by rinsing with water or by washing the keratin fibers with a shampoo.

In an embodiment, it is described a method for increasing the elasticity of keratin fibers comprising the steps of:
(i) treating the keratin fibers by topical application of a cosmetic composition comprising, in a cosmetically acceptable medium:
   (a) a mixture of Hydroxypropylgluconamide and Hydroxypropylammonium Gluconate; and
   (b) lactic acid, or any cosmetically acceptable salt thereof;
(ii) allowing the composition to remain in contact with the keratin fibers for a period ranging from about 5 seconds to about 1 hour;
(iiib) removing the cosmetic composition from the keratin fibers by washing the keratin fibers with a shampoo; and
(iv) rinsing the keratin fibers with water.

In an embodiment, it is described a method for increasing the elasticity of keratin fibers comprising the steps of:
(i) treating the keratin fibers by topical application of a cosmetic composition comprising, in a cosmetically acceptable medium:
   (a) a mixture of Hydroxypropylgluconamide and Hydroxypropylammonium Gluconate; and
   (b) lactic acid, or any cosmetically acceptable salt thereof;
(ii) allowing the composition to remain in contact with the keratin fibers for a period ranging from about 10 seconds to 45 minutes;
(iiib) removing the cosmetic composition from the keratin fibers by washing the keratin fibers with a shampoo; and
(iv) rinsing the keratin fibers with water.

In an embodiment, it is described a method for increasing the elasticity of keratin fibers comprising the steps of:
(i) treating the keratin fibers by topical application of a cosmetic composition comprising, in a cosmetically acceptable medium:
   (a) a mixture of Hydroxypropylgluconamide and Hydroxypropylammonium Gluconate;
   (b) lactic acid, or any cosmetically acceptable salt thereof; and
   (c) optionally, maleic acid, or any cosmetically acceptable salt thereof;
(ii) allowing the composition to remain in contact with the keratin fibers for a period ranging from about 5 seconds to about 1 hour (60 minutes).

In an embodiment, the cosmetic use of a composition as defined above to increase the elasticity of keratin fibers is disclosed.

In an embodiment, it is disclosed the cosmetic use of a composition comprising, in a cosmetically acceptable medium:
(a) a mixture of Hydroxypropylgluconamide and Hydroxypropylammonium Gluconate; and
(b) lactic acid, or any cosmetically acceptable salt thereof;
for increasing the elasticity of keratin fibers.

In an embodiment, it is disclosed the cosmetic use of a composition comprising, in a cosmetically acceptable medium:
(a) a mixture of Hydroxypropylgluconamide and Hydroxypropylammonium Gluconate; and
(b) lactic acid, or any cosmetically acceptable salt thereof;
for increasing the elasticity of human hair.

In an embodiment, it is disclosed the cosmetic use of a composition comprising, in a cosmetically acceptable medium:
(a) a mixture of Hydroxypropylgluconamide and Hydroxypropylammonium Gluconate; and
(b) lactic acid, or any cosmetically acceptable salt thereof;
for increasing the elasticity of human hair, which has been previously damaged as a result of at least one treatment selected from hair bleaching, hair dyeing, hair straightening, hair permanent wave (hair perm), blow-drying, or combinations thereof.

In an embodiment, it is disclosed the cosmetic use of a composition comprising, in a cosmetically acceptable medium:
(a) a mixture of Hydroxypropylgluconamide and Hydroxypropylammonium Gluconate; and
(b) lactic acid, or any cosmetically acceptable salt thereof;
for increasing the elasticity of human hair, which has been previously damaged as a result of at least one treatment selected from hair bleaching, hair dyeing or combinations thereof.

The following examples are given in order to provide a person skilled in the art with a sufficiently clear and complete explanation of the present disclosure but should not be considered as limiting of the essential aspects of its subject, as set out in the preceding portions of this description.

All percentages, ratios and proportions herein are by weight, unless otherwise specified. All molecular weights herein are weight average molecular weights, unless otherwise specified. All temperatures are in degrees Celsius (°C) unless otherwise specified.

Ingredients are identified by chemical or INCI name, or otherwise defined below.

### EXAMPLES

### 1. Preparation of the compositions

The compositions as shown in Table 1 were prepared by mixing and stirring until complete homogenization in a kettle. Composition C1 is a composition according to the present disclosure. Compositions CC1 and CC2 are comparative compositions.

**Table 1 - Compositions (wt.% as active matter based on the total weight of the composition)**

| Ingredients (INCI name) | Compositions | | |
|---|---|---|---|
| | C1 | CC1 | CC2 |
| L-Lactic Acid | 1.2 | 1.2 | --- |
| Aqueous mixture of Hydroxypropyl Gluconamide (and) Hydroxypropyl Ammonium Gluconate* | 0.5 | --- | 0.5 |
| Aqua (Water (Eau)) | Up to 100 | Up to 100 | Up to 100 |

| | | | |
|---|---|---|---|
| * FiberHance^{™} bm solution marketed by Ashland Inc (active matter about 50 wt.%) | | | |

### 2. Application of the compositions to keratin fibers

### 2.1. Pre-wash

Swatches of virgin dark wavy/curly Caucasian hair were selected.
24 hours prior to the treatment, the strands were washed with shampoo and rinsed under tap water according to the following process:
- the swatches were soaked for 1 hour in a solution of 10 wt.% Pro You^{™} Neutral Shampoo (commercially available from Revlon Professional) and tap water;
- the swatches were rinsed for 60 seconds under tap water;
- excess water was removed with a towel; and
- finally, the swatches were dried for 15 minutes at low temperature using a Pro Iron Dryer 3000 City manufactured by Tectools.

### 2.2. Damaging (bleaching) hair

The swatches of hair of step 2.1 were damaged with a bleach mark to be used as reference for measuring purposes, the bleach mark being located at the same position (approximately at 1/3 of the hair swatches) according to the following process:
- a powder bleaching composition (Revlon Professional Magnet^{™} Blondes Ultimate Powder 9, commercially available) was mixed with a 9% by weight aqueous hydrogen peroxide composition (Revlon Professional Magnet^{™} Blondes Ultimate Oil Developer, commercially available) in a non-metallic bowl at a ratio 1:2 (powder: hydrogen peroxide) until a homogeneous mixture was obtained;
- the swatches were soaked with a brush with the mixture prepared above thoroughly and uniformly to coat the swatches;
- the swatches were wrapped in aluminum foil and were allowed to stand at 25°C for a period of 30 minutes;
- the swatches were rinsed for 1 minute under tap water.

### 2.3. Application

The composition C1 and the comparative compositions CC1-CC2 of Ex. 1 were applied to the swatches damaged as described in point 2.2 above, according to the following process:
- the swatches were soaked for 5 minutes in 40 g of the corresponding compositions (C1, CC1 or CC2);
- the swatches were rinsed for 20 seconds under tap water;
- the swatches were soaked for 30 seconds in a 200 mL solution of 10 wt.% Revlon Professional Pro You^{™} Neutral Shampoo (commercially available) and tap water, under agitation (about 1000 rpm);
- the swatches were rinsed for 30 seconds under tap water;
- excess water was removed with a towel; and
- finally, the swatches were dried for 5 minutes at low temperature using a Pro Iron Dryer 3000 City manufactured by Tectools.

Step 2.1 to 2.3 were repeated 4 additional times (5 in total) to replicate hair that has been seriously damaged (overprocessed by bleaching).

### 3. Evaluation of hair elasticity

Once the hair swatches were dry, their length was determined using a measured graph paper (in centimeters) (initial length).

Hair swatches were stretched from the roots to the ends until they can no longer be stretched. Their length was determined again (length during stretching).

The force was maintained for 10 seconds by placing two 100 g weights at the roots and ends of the swatches.

Finally, the weights were removed, and the length of the swatches was determined again (considering approximately 70% of the strands of each swatch) (final length).

For each hair swatch, the final length was normalized by the length of a hair swatch to which no treatment was applied.

The hair elasticity, expressed as % recovered elasticity, was calculated as follows: $\begin{matrix}% recovered \\ elasticity\end{matrix} = \frac{Final length \left(cm\right) - Initial length \left(cm\right)}{Length during stretching \left(cm\right) - Initial length \left(cm\right)} \times 100$

The results are shown in Table 2. Each value represents the mean of 3 hair swatches coming from different donors.

**Table 2 - % recovered elasticity**

| Composition | % of recovered elasticity |
|---|---|
| C1 | 25.77 |
| CC1 | 10.65 |
| CC2 | 10.34 |

From the experimental results, it can be concluded that compositions of the present disclosure (Composition C1) are effective in producing noticeable increases in hair elasticity (expressed as % recovered elasticity) compared to Comparative Compositions CC1 and CC2.

Modifications, which do not affect, alter, change or modify the essential aspects of the compositions and methods described above, are included within the scope of the present disclosure.

## Claims

1. A method for increasing the elasticity of keratin fibers comprising the steps of:
(i) treating the keratin fibers by topical application of a cosmetic composition comprising, in a cosmetically acceptable medium:
(a) a mixture of Hydroxypropylgluconamide and Hydroxypropylammonium Gluconate; and
(b) lactic acid, or any cosmetically acceptable salt thereof;
(ii) allowing the composition to remain in contact with the keratin fibers for a period ranging from about 5 seconds to about 1 hour.

2. The method according to claim 1, wherein in step (ii) the composition is allowed to remain in contact with the keratin fibers for a period ranging from about 10 seconds to about 45 minutes.

3. The method according to claim 1 or claim 2, wherein in the cosmetic composition the molar ratio of Hydroxypropylgluconamide to Hydroxypropylammonium Gluconate in component (a) ranges from about 20:80 to about 80:20.

4. The method according to any one of claims 1 to 3, wherein in the cosmetic composition the component (b) comprises from about 60% to about 100% of L-Lactic acid, with respect to the total lactic acid content, or any cosmetically acceptable salt thereof.

5. The method according to any one of claims 1 to 4, wherein in the cosmetic composition the total amount of the component (a) is in the range of about 0.05 to about 10 wt.%, based on the total weight of the composition.

6. The method according to any one of claims 1 to 5, wherein in the cosmetic composition the total amount of the component (b) is in the range of about 0.2 to about 10 wt.%, based on the total weight of the composition.

7. The method according to claim 1, wherein the cosmetic composition comprises, in a cosmetically acceptable medium, based on the total weight of the composition:
(a) about 0.05-10 wt.% of a mixture of Hydroxypropylgluconamide and Hydroxypropylammonium Gluconate; and
(b) about 0.2-10 wt.% of lactic acid, or any cosmetically acceptable salt thereof.

8. The method according to claim 7, wherein the cosmetic composition comprises, in a cosmetically acceptable medium, based on the total weight of the composition.
(a) about 0.1-7.5 wt.% of a mixture of Hydroxypropylgluconamide and Hydroxypropylammonium Gluconate; and
(b) about 0.3-7.5 wt.% of lactic acid, or any cosmetically acceptable salt thereof.

9. The method according to claim 8, wherein the cosmetic composition comprises, in a cosmetically acceptable medium, based on the total weight of the composition.
(a) about 0.2-7.5 wt.% of a mixture of Hydroxypropylgluconamide and Hydroxypropylammonium Gluconate, wherein the molar ratio of Hydroxypropylgluconamide to Hydroxypropylammonium Gluconate in component (a) ranges from about 20:80 to about 80:20; and
(b) about 0.5-7.5 wt.% of L-lactic acid, or any cosmetically acceptable salt thereof.

10. The method according to any one of claims 1 to 9, wherein the keratin fibers are human hair.

11. The method according to claim 10, wherein human hair has been previously damaged as a result of at least one treatment selected from hair bleaching, hair dyeing, hair straightening, hair permanent wave (hair perm), blow-drying, or combinations thereof.

12. The method according to any one of claims 1 to 11, wherein the cosmetic composition has a pH in the range from about 3.0 to about 6.5, measured at 25°C under atmospheric conditions.

13. The method according to any one of claims 1 to 12, additionally comprising step (iii) removing the cosmetic composition from the keratin fibers by rinsing with water or by washing the keratin fibers with a shampoo.
